# EUROPEAN PATENT SPECIFICATION

(11) **EP 2 691 569 B1**
(45) Date of publication and mention of the grant of the patent: **07.01.2015**
(21) Application number: 12720992.2
(22) Date of filing: 30.03.2012
(51) Int. Cl.: D06M 11/56, D06M 11/66, D06M 15/03, D06M 16/00, D06M 23/10, A61K 9/70, C11D 7/08, C11D 7/10, C08L 5/16, C09D 105/16, C11D 3/22, C09J 105/16

(54) **BINDER AND PROCESS FOR PRODUCING FABRICS CONTAINING CYCLODEXTRINS FIXED BY SAID BINDER**
BINDEMITTEL UND VERFAHREN ZUR HERSTELLUNG EINES TEXTILEN STOFFS ENTHALTEND MIT DIESEM BINDEMITTEL FIXIERTE CYCLODEXTRINE
LIANT ET PROCÉDÉ DE PRODUCTION D'UN TEXTILE CONTENANT DES CYCLODEXTRINES FIXÉES PAR CE LIANT

(30) Priority: 31.03.2011 IT RM20110166
(43) Date of publication of application: 05.02.2014
(73) Proprietor: Gianis S.r.l., 59100 Prato PO (IT)
(72) Inventor: TRAVAGLINI Nicola, 59100 Prato Po (IT); NISTRI Giampaolo, 59100 Prato Po (IT)
(74) Representative: Perronace, Andrea
(86) International application number: PCT/IT2012/000096
(87) International publication number: WO 2012/131745

(56) References cited:
- FR-A1- 2 910 481
- US-A1- 2005 260 905

## Description

The present invention concerns a binder and a process of production of fabrics containing cyclodextrins fixed by said binder.

More precisely, the present invention permits to fix a binder to textile fibers, natural, synthetic or artificial fibers and, by means of thermal processes, to fix to the same binder the cyclodextrins so as to generate fabrics able to incorporate active principles and to release them along the time. The invention peculiarities consist in: the production of functionalized fabrics, adapted to be loaded with molecules of interest and to release them gradually along the time, avoiding the alteration of fabric original characteristics; the possibility to repeat several times the loading of such molecules, so as to define such fabrics as "reloadable" or "rechargeable"; the identification of a binder that interacts with different types of fabrics, independently from their composition; the identification of a binder able to interact with several types of cyclodextrins.

### STATE OF ART

As is well known, cyclodextrins are cyclic oligosaccharides that are generated by the enzymatic hydrolysis of the starch. After this reaction, oligomeric units are formed which are constituted by 6, 7 or 8 glucopyranoside molecules that close themselves in a ring, generating a particular frusto-conical form.

These ring-structure molecules, with a hydrophilic external structure and a hydrophobic internal cavity, are able to encapsulate or complex a large range of molecules of different nature, modifying their chemical-physical properties and permitting the release along the time in a controlled manner.

The interaction between molecule and cyclodextrin can take place both by inclusion in the internal cavity and by electrochemical affinity outside the molecule itself. The so formed complex is in a dynamic state in which there is a continuous variation of the equilibrium between the free and the complex forms, that results in a gradual and controlled release of the molecule from the complex.

The cyclodextrins, due to their peculiar ability to form such complexes, have found large applications in pharmaceutical, cosmetic and food fields and in the detergent products sector.

The possibilities of cyclodextrins applications have been furthermore extended thanks to the availability of several types of derived and functionalized cyclodextrins on the market, in which the introduction of specific substitutes has permitted to suitably modify their characteristics, increasing or decreasing, depending on the case, their solubility in water, complexing ability, efficacy to solubilize, chemical stability, etc.

The cyclodextrins represent nowadays an important group of textile auxiliaries thanks to their characteristics of biodegradability, non-toxicity, and large versatility of application.

Substrates of textile fibers, in particular fibers and fabrics, containing incorporated cyclodextrins, able to complex and release in a controller manner active principles such as pharmaceuticals, pheromones, cosmetic substances, perfumes, etc., are yet well known in literature and different processes for their production are also well known.

Thus, for example, a process for the incorporation of a cyclodextrin in a fiber or in a fiber-based material is known, which consists in heating, at a temperature value in the range of 150 to 220°C, a material or a fiber whereon a solid mixture of cyclodextrins or related complex, a poly-carboxylic acid, an anhydride of the poly-carboxylic acid and, eventually, a catalyzer has been applied (WO-A-00/47811).

This process, even if it is of simple realization, has the drawback of negatively influencing the softness and the elasticity of the treated fabrics. Moreover, such a process can be applied only to the support whose fibers resist to these temperatures or do not undergo degradation phenomena.

Patent document US20050260905 relates to a textile material with a cyclodextrin finishing having a polymeric matrix containing cyclodextrin and/or a cyclodextrin derivative, as well as a method for the manufacture of such a textile material.

The German patent application DE-A-19810951 provides the binding of cyclodextrins to the fiber by means of polymeric chains that act as bridges. This process has the drawback of limiting the cyclodextrins application to materials or fibers that allow to bind themselves to said polymeric chains. Moreover, possible interactions of such polymeric chains with the functional groups of the cyclodextrins can alter their ability to bind and incorporate the active principles by neutralizing as a consequence their efficacy to complex molecules.

The Italian patent application VR2001A000114 describes a process for the production of a substrate containing incorporated cyclodextrins, which is able to absorb and release in a controlled manner the active principles. Even this process, that uses the ability of the cyclodextrins to be incorporated in polymeric matrices and to create with the latter new solid structure with the substrate, is not devoid of disadvantages. Its application, indeed, is limited only to the polymeric matrices having such characteristics. In addition, the polymeric matrices can interact with the functional groups of the cyclodextrins diminishing, as consequence, their ability to bind and incorporate the active principles.

It has been demonstrated that it is possible to fix cyclodextrins on synthetic or natural fibers in a physical or chemical way by utilizing cyclodextrins with an alkyl chain or other hydrophobic substituents (DE-A-4035378). The chain moves inside the fibers while cyclodextrin remains on the fiber surface, owing to the fact that the cyclodextrin has hydrophilic properties outside itself. In addition, it is possible to chemically fix cyclodextrins by reaction of their functional groups with the fibers ones. For example, if the cyclodextrin had an acid group, it could be possible, in analogy with acid dyes, to fix it on polyamide fibers. For the anchorage on wool fibers, cyclodextrins are used with a suitable reactive group.

Moreover, the cyclodextrins fixing on cellulose fibers using the epichlorhydrin as cross-linking agent has been described (US Patent 4,357,468). Nevertheless, examples of cyclodextrins fixing are always described as made on textile materials containing reactive functional groups. Very different is the case of polypropylene which, having no reactive or charged functional groups, can set up interaction forms very difficultly. Few examples are reported which concern the fixing of cyclodextrin to such fabric, among which there are methods based on electron beams or microwaves irradiation technologies (Le Thuaut, 1999; Mirabedini et al., 2004).

Patent document FR2910481 discloses a sizing composition for mineral fiber fibres comprising a phenolic resin, urea, a crosslinking catalyst, possibly additives, characterized in that the crosslinking catalyst is a mixture of ammonium sulfamate and ammonium sulfate. The document also concerns a heat or sound insulation product based on mineral fibers, especially glass fibers or rock wool, where the fibers are coated with a size composition according to the document.

A further problem of the known techniques is the resistance of the functionalization to washing, that is claimed only in the cases in which there is a real chemical fixing of the cyclodextrin to the fabric or anyway when the interaction of the cyclodextrin with the fabric is carried out in rather drastic temperature and/or pH conditions, with consequent possible loss of the fabric original qualities.

### OBJECT AND SUBJECT MATTER OF THE INVENTION

Object of the present invention is to provide a binder to be used on different type of fabrics, independently on their nature, so as to be able to load cyclodextrins or their derivatives on fabrics, that solves the problems and overcomes the drawbacks of the known techniques. Indeed, an advantage of the present invention consists in the fact that the binder is able to favor the cyclodextrin interaction with different types of fabrics including the poly-propylene. A further advantage of the present invention is that the binder allows the binding of several types of cyclodextrins, without limiting itself to only one type as in the case of the well-known techniques.

It is further specific object of the present invention to provide a process for the production of a fabric which is able to absorb and release an active principle, and which is reloadable and therefore allows to repeat more than one time the operation of absorption and release of the active principle.

An advantage of the invention process is the possibility of using quite soft reaction conditions for the functionalization of the fabrics that are such not to modify the original properties but favoring anyway the functionalization with a longer resistance to washing.

It is further specific object of the present invention a fabric produced utilizing the process that is object of the invention and apt to be loaded with active principles or other substances of different nature and action and to gradually release them along time.

It is further specific object of the present invention the fabric object of the invention, which is loaded with said active principles.

It is subject-matter of the present invention a binder composition for binding cyclodextrins to fabrics, characterized in that it is a liquid mixture including sulfamic acid with a weight percentage comprised between 0.8 and 1.2 over said liquid mixture, ammonium sulphamate with weight percentage comprised between 1.2 and 1.6 over said liquid mixture, and ammonium sulfate with weight percentage comprised between 2.8 and 3.2 over said liquid mixture.

Preferably according to the invention, the sulfamic acid is present in a weight percentage comprised between 0.9 and 1.1 over the whole mixture, the ammonium sulphamate is present in a weight percentage comprised between 1.3 and 1.5, and the ammonium sulfate is present in a weight percentage comprised between 2.9 and 3.1 over the whole mixture.

Preferably according to the invention, the sulfamic acid being present in a weight percentage substantially equal to 1.0 over the whole mixture, the ammonium sulphamate being present in a weight percentage substantially equal to 1.5, and the ammonium sulfate being present in a weight percentage substantially equal to 3.0 over the whole mixture.

Preferably according to the invention, the mixture comprises water as solvent.

Preferably according to the invention, said liquid mixture comprises acids and salts constituted by sulfamic acid, ammonium sulphamate and ammonium sulfate.

It is specific subject-matter of the present invention a process for the production of a fabric suitable to absorb and release an active principle, characterised in that it uses a binder and at least a cyclodextrin and/or a derivative thereof, the binder being the binder as defined in any claim 1 to 5, the process being further characterised in that it comprises the following steps:
A. placing the fabric in a bath with a weight/volume of the fabric/binder not larger than 1/10 and a temperature comprised in the range 30-80°C;
B. placing in the bath at least a cyclodextrin and/or a derivative thereof, and agitating constantly such mixture for a time larger than 60 minutes at the same temperature of step A;
C. discharging the bath and rinsing the fabric with cold water;
said time and temperature depending on the composition of the fibers constituting the fabric and the type of the cyclodextrin.

Preferably according to the invention, said weight/volume of the fabric/binder is comprised between 1/10 and 1/15.

Preferably according to the invention, said temperature is comprised between 60 and 80 ° C, more preferably between 65 and 75 °C.

Preferably according to the invention, at least a cyclodextrin and/or a derivative thereof is present with a concentration comprised between 20 and 40% in a suitable solvent, preferably between 25 and 35%, said suitable solvent being for example dimethyl sulfoxide.

Preferably according to the invention, in step B said time is comprised between 60 and 120 minutes, preferably between 80 and 100 minutes.

Preferably according to the invention, in step B said temperature is equal or larger than 50°C.

It is specific subject-matter of the present invention a fabric suitable to absorb and release an active principle, characterised in that it is produced by utilizing the binder as defined in the subject-matter of the invention, and performing the process steps as defined by the invention.

It is specific subject-matter of the present invention a fabric having an active principle ready to be released in a gradual and controller way, characterised in that it is the fabric according to the subject-matter of the invention, treated by any of the following processes:
- placing the active principle in a bath at ambient temperature and maintaining it in the bath with slight agitation during a time interval not smaller than 30 minutes, followed by drying; or
- Atomization of the solution with the active principle onto the fabric, waiting few minutes for its absorbing.

It is specific subject-matter of the present invention a use of the fabric according to the subject-matter of the invention, for the controlled release of active principles.

Preferably according to the invention, the use is the intra-dermal controlled release of active principles.

The invention will be now described by way of illustration but not by way of limitation, making reference to some preferred embodiments.

The binder of the invention is used to pre-treat the fabrics.

Once treated, the binder remains on the fabrics themselves, favoring the interactions with the cyclodextrin, that is therefore bound to the fabric in a durable manner.

The cyclodextrins, in turn, is able to absorb active principles that it will release later on in a gradual and controlled manner.

Such fabrics, once utilized, can be washed and loaded again with the active principles by means of a simple bath. The reloading process can be repeated many times, since the fabrics remains functionalized after the washing, due to the fact that both the binder and the cyclodextrin remains on the fabric.

According to the present invention, with "binder" is meant every substance that is able to bind itself to the fabric favoring its interaction with the cyclodextrin and is able to interact itself with the cyclodextrin. The interaction forces involved can be Van Deer Waals forces, electrostatic forces, ionic bonds, H-bonds, covalent bonds.

According to a particular embodiment, the binder is essentially but not exclusively constituted by a mixture of ammonium inorganic salts and inorganic acids.

To the knowledge of the inventors, such substances do not act per se properly as binder, but they function as softening agent of the fabric, giving it weak hydrophilic properties and favoring the trapping of the cyclodextrin in the fabric mesh.

Always according to a particular embodiment of the present invention, such effect of softening is used in conjunction with that of the bath temperature in order to fix the binder to maximize the cyclodextrin interaction with the fabric so that the cyclodextrin remains bound to it for long time.

In accordance with the present invention, the utilizable cyclodextrins can be both natural and derived, both neutral and cationic or anionic, both polymerized and functionalized, as such or as mixture thereof.

In a particular embodiment, a functionalised derivative of the beta-cyclodextrin is used.

According to the invention, the loadable substances can be of different nature, for example drugs, pheromones, integrators, beauty substances, perfumes, essential oils; insect-repellent substances, bactericides can be incorporated as well; the fabrics can also acquire antismog functions. Therefore, the fabric can be used with both with intra-dermal aim or with other aims.

In the present invention, the following are considered as textiles: fabrics, non-woven fabrics, tubular fabrics with seams or without seams, felts, waddings, tops, yarns, garments, linings, polymeric supports, film polyurethanes.

According to the invention, the process of application can be applied in tumblers, dyeing jet machine, dyeing machine in general, washing basket, and can be integrated in processes of dyeing or purging, bleaching, felting. The process for the production of functionalized and loaded textiles consists in three steps described in the following:

In the first step there is the fixation of the binder to the fibers, inserting in the machine the textile together with the binder, in appropriate weight textile/bath ratio and at a suitable temperature value.

In the second step there is the fixation of the cyclodextrin, inserting in the bath the cyclodextrin under constant agitation for an opportune time span and at a suitable temperature value as a function of the composition of the fibers constituting the textile and the type of utilized cyclodextrin. At the end of this step, the bath is discharged and a rinsing has been performed in cold water.

The third step provides the introduction of the active principle in the textile following two different alternative possible techniques:
- insertion of the active principle in a bath at room temperature under moderate agitation for a suitable time, followed by drying;
- nebulization of the active principle solution onto the textile, waiting few minutes for its adsorption.

### DESCRIPTION OF A PARTICULAR EMBODIMENT

In the following a particular embodiment of the present invention is described by way of illustration but not by way of limitation.

According to the invention process, the binder is fixed to the textile by insertion of both ones in the machine, in a weight/volume textile/binder ratio of 1/10 and at a temperature value of 70°C (the temperature should be equal or larger than 30 °C, preferably equal or larger than 50 °, and in any case it cannot go beyond 100°C, advantageously if below 80°C).

The binder, according to this particolar embodiment, is constituted by a liquid mixture comprising a solvent (conveniently water, or even e.g. dimethyl-sulfoxide) and: sulfamic acid, ammonium sulphamate and ammonium sulfate in weight ratios 1 : 1,5 : 3.

In the present description, one indicates by X:Y:Z the weight percentages, over the whole mixture, of the three essential components of the same mixture, i.e. the sulfamic acid has a weight percentage equal to (approximately) X, the ammonium sulphamate equal to (approximately) Y and the ammonium sulfate equal to (approximately) Z over the whole mixture. If these percentages vary in a range, the synthetic notation X₁-X₂:Y₁-Y₂:Z₁-Z₂ will be used, therefore the sulfamic acid has a weight percentage between (approximately) X₁ and (approximately) X₂, the ammonium sulphamate has a weight percentage between (approximately) Y₁ and (approximately) Y₂ and the ammonium sulfate has a weight percentage between (approximately) Z₁ and (approximately) Z₂ over the whole mixture.

Still according to this particular embodiment, the textile is constituted by polypropylene at 90% together with 10 % of elastomer.

The solution utilized in this case is the dimethyl-sulfoxide.

Afterwards, the cyclodextrin is fixed, by introducing it in a bath under constant agitation at a temperature of 70°C, for a time period of 90 min, followed by a washing in cold water.

In this particular embodiment, an allyl derivative of the betacyclodextrin is used.

After that the textile functionalization is completed, the active principle to be released is introduced by means of its insertion in the bath at room temperature under moderate agitation for 40 min, followed by drying.

In general, the period of the baths can vary depending on the fiber and on the results to be achieved.

In the present embodiment, a mixture of integrator has been used as model of possible substances with which the textile is to be reloaded.

According to this embodiment, trials have been performed in order to evaluate the effective capacity of the textile functionalization.

Trials have been also conducted to evaluate the charging capacity of the integrator mixture by the textile functionalized according to the invention.

In addition, trials have been conducted to evaluate the capacity of the textile to maintain the functionalization after several washings and consequently to verify the effective capacity of recharging of the textile.

The following table summarizes the results obtained by the trials repeated three times, expressed as percentage of each component remained bound at the textile with respect to the initial introduced amount.

| EVALUATION OF THE TEXTILE FUNCTIONALIZATION CAPACITY | |
|---|---|
| Binder bound to the textile | 29.0±3% |
| Cyclodextrin bound to the textile | 6.4±0,6% |

| EVALUATION OF THE RECHARGING CAPACITY OF THE TEXTILE FUNCTIONALIZED ACCORDING TO THE INVENTION | |
|---|---|
| Integrators bound to the textile functionalized according to the invention | 74.8±1,7% |

| EVALUATION OF THE TEXTILE CAPACITY TO MAINTAIN THE FUNCTIONALIZATION FOR THE RELOAD | |
|---|---|
| Cyclodextrin remained bound to the textile after the first washing | 3.9±0,8% |
| Integrators bound to the textile functionalized after a reload following the first washing | 75.2±1,2% |
| Cyclodextrin remained bound to the textile after three washings | 3.1±0,2% |
| Integrators bound to the textile functionalized after a reload following three washings | 74.5±1,1% |

The decrease of the cyclodextrins after the first washing is due to the elimination of the cyclodextrins that remain on the surface during the step of their insertion and are almost all eliminated during the washing.

The results demonstrate the binder capacity to allow a textile functionalization in rather weak conditions.

The results also demonstrate the high charging capacity of the textile functionalized according to the invention. In fact, it is able to bind about the 75% of the integrators mixture introduced in the bath.

It has been as well demonstrated the textile capacity to maintain a high level of functionalization even after washings. Indeed, after the first washing the integrators amount that finds itself on the textile after the reload is always about 75%.

Furthermore, after the third washing, a cyclodextrin amount remains bound to the fabric that is such to allow additional reloads with the integrators.

So, according to the embodiment previously described, it is correct to define the textile, functionalized according to the invention, as textile with gradual release of active principles, reloadable in domestic environment.

### DESCRIPTION OF ADDITIONAL EMBODIMENTS

All the below-listed fibers have been examined in 100% o in combination with 5% of elastomer, obtaining the same results. The binder composition is as in the previous embodiment, only weight ratios are changed. The temperature and the application time refer to the cyclodextrins application, see previous example.

| | |
|---|---|
| Wool: | binder composition 0,8 : 1,6 : 3,1; temperature 65°C; application time 60 minutes. |
| Cotton: | binder composition 1 : 1,4 : 3,1; temperature 65 or; application time 60 minutes. |
| Nylon: | binder composition 1 : 1,5 : 3; temperature 70°C; application time 90 minutes. |
| Polyester: | binder composition 1 : 1,3 : 3,2; temperature 80°C; application time 120 minutes. |
| Viscose: | binder composition 1.2 : 1.2 : 2.8; temperature 60 °C; application time 60 minutes. |
| Acrilico/lycra: | binder composition 1.1 : 1.2 : 2.8; temperature 70 °C; application time 70 minutes. |

Such realizations have provided a functionality result comparable to the polypropylene (demonstrated in laboratory by means of a colored reagent) since the binder is able to bind itself to all fibers with the previously described composition variation and within the functionality range.

The applications field of the present invention can be: textile, pharmaceutical, cosmetic, veterinary, agricultural, nautical, building and furniture industries, shoes and clothes manufacturing, anti-accident industry.

In the foregoing preferred embodiments have been described, however it is to be intended that those skilled in the art can modify them without departing from the relevant scope of protection, as defined by the enclosed claims.

## Claims

1. Binder composition for binding cyclodextrins to fabrics, **characterized in that** it is a liquid mixture including sulfamic acid with a weight percentage comprised between 0.8 and 1.2 over said liquid mixture, ammonium sulphamate with weight percentage comprised between 1.2 and 1.6 over said liquid mixture, and ammonium sulfate with weight percentage comprised between 2.8 and 3.2 over said liquid mixture.

2. Composition according to claim 1, **characterised in that** the sulfamic acid is present in a weight percentage comprised between 0.9 and 1.1 over the whole mixture, the ammonium sulphamate is present in a weight percentage comprised between 1.3 and 1.5, and the ammonium sulfate is present in a weight,percentage comprised between 2.9 and 3.1 over the whole mixture.

3. Composition according to claim 2, **characterised in that** the sulfamic acid being present in a weight percentage substantially equal to 1.0 over the whole mixture, the ammonium sulphamate being present in a weight percentage substantially equal to 1.5, and the ammonium sulfate being present in a weight percentage substantially equal to 3.0 over the whole mixture.

4. Composition according to any claim 1 to 3, **characterized in that** the mixture comprises water as solvent.

5. Composition according to any claim 1 to 4, **characterized in that** said liquid mixture comprises acids and salts constituted by sulfamic acid, ammonium sulphamate and ammonium sulfate.

6. Process for the production of a fabric suitable to absorb and release an active principle, **characterised in that** it uses a binder and at least a cyclodextrin and/or a derivative thereof, the binder being the binder as defined in any claim 1 to 5, the process being further **characterised in that** it comprises the following steps:
A. placing the fabric in a bath with a weight/volume of the fabric/binder not larger than 1/10 and a temperature comprised in the range 30-80°C;
B. placing in the bath at least a cyclodextrin and/or a derivative thereof, and agitating constantly such mixture for a time larger than 60 minutes at the same temperature of step A;
C. discharging the bath and rinsing the fabric with cold water;
said time and temperature depending on the composition of the fibers constituting the fabric and the type of the cyclodextrin.

7. Process according to claim 6, **characterised in that** said weight/volume of the fabric/binder is comprised between 1/10 and 1/15.

8. Process according to claim 6 or 7, **characterised in that** said temperature is comprised between 60 and 80° C, more preferably between 65 and 75°C.

9. Process according to any claim 6 to 8, **characterised in that** at least a cyclodextrin and/or a derivative thereof is present with a concentration comprised between 20 and 40% in a suitable solvent, preferably between 25 and 35%, said suitable solvent being for example dimethyl sulfoxide.

10. Process according to any claim 6 to 9, **characterised in that** in step B said time is comprised between 60 and 120 minutes, preferably between 80 and 100 minutes.

11. Process according to any claim 6 to 10, **characterised in that** in step B said temperature is equal or larger than 50°C.

12. Fabric suitable to absorb and release an active principle, **characterised in that** it is produced by utilizing the binder as defined in any claim 1 to 5, and performing the process steps as defined by any claim 6 to 11.

13. Fabric having an active principle ready to be released in a gradual and controller way, **characterised in that** it is the fabric according to claim 12, treated by any of the following processes:
- placing the active principle in a bath at ambient temperature and maintaining it in the bath with slight agitation during a time interval not smaller than 30 minutes, followed by drying; or
- Atomization of the solution with the active principle onto the fabric, waiting few minutes for its absorbing.

14. Use of the fabric according to claim 12 or 13, for the controlled release of active principles.

15. Use of the fabric according to claim 14, for intra-dermal controlled release of active principles.

## Patentansprüche

1. Bindemittel-Zusammensetzung zur Bindung von Cyclodextrinen an Textilgewebe, **dadurch gekennzeichnet, dass** es sich um eine flüssige Mischung handelt, welche Sulfaminsäure mit einem Gewichtsprozentsatz zwischen 0,8 und 1,2, bezogen auf die flüssige Mischung, Ammoniumsulfamat mit einem Gewichtsprozentsatz zwischen 1,2 und 1,6, bezogen auf die flüssige Mischung, und Ammoniumsulfat mit einem Gewichtsprozentsatz zwischen 2,8 und 3,2, bezogen auf die flüssige Mischung, umfasst.

2. Zusammensetzung nach Anspruch 1, **dadurch gekennzeichnet, dass** die Sulfaminsäure in einem Gewichtsprozentsatz zwischen 0,9 und 1,1, bezogen auf die Gesamtmischung, vorliegt, das Ammoniumsulfamat in einem Gewichtsprozentsatz zwischen 1,3 und 1,5 vorliegt und das Ammoniumsulfat in einem Gewichtsprozentsatz zwischen 2,9 und 3,1, bezogen auf die Gesamtmischung, vorliegt.

3. Zusammensetzung nach Anspruch 2, **dadurch gekennzeichnet, dass** die Sulfaminsäure in einem Gewichtsprozentsatz, der im wesentlichen gleich 1,0 ist, bezogen auf die Gesamtmischung, vorliegt, das Ammoniumsulfamat in einem Gewichtsprozentsatz, der im wesentlichen gleich 1,5 ist, vorliegt und das Ammoniumsulfat in einem Gewichtsprozentsatz, der im wesentlichen gleich 3,0 ist, bezogen auf die Gesamtmischung, vorliegt.

4. Zusammensetzung nach irgendeinem der Ansprüche 1 bis 3, **dadurch gekennzeichnet, dass** die Mischung Wasser als Lösungsmittel umfasst.

5. Zusammensetzung nach irgendeinem der Ansprüche 1 bis 4, **dadurch gekennzeichnet, dass** die flüssige Mischung Säuren und Salze, gebildet von Sulfaminsäure, Ammoniumsulfamat und Ammoniumsulfat, umfasst.

6. Verfahren zur Herstellung eines Textilgewebes, das zur Absorption und Freisetzung eines Wirkstoffs geeignet ist, **dadurch gekennzeichnet, dass** ein Bindemittel und mindestens ein Cyclodextrin und/oder ein Derivat davon verwendet wird, wobei das Bindemittel das Bindemittel nach irgendeinem der Ansprüche 1 bis 5 ist und das Verfahren ferner **dadurch gekennzeichnet ist, dass** es die folgenden Schritte umfasst:
A) Platzieren des Textilgewebes in ein Bad mit einem Gewicht/Volumen-Verhältnis des Textilgewebes/Bindemittels von nicht mehr als 1/10 und einer Temperatur, die im Bereich von 30-80°C liegt;
B) Platzieren von mindestens einem Cyclodextrin und/oder einem Derivat davon in das Bad, und konstantes mechanisches Bewegen einer solchen Mischung für einen längeren Zeitraum als 60 Minuten bei der gleichen Temperatur von Schritt A;
C) Entleeren des Bades und Spülen des Textilgewebes mit kaltem Wasser;
wobei die Zeit und Temperatur von der Zusammensetzung der Fasern, welche das Textilgewebe ausmachen, sowie dem Cyclodextrin-Typ abhängen.

7. Verfahren nach Anspruch 6, **dadurch gekennzeichnet, dass** das Gewicht/Volumen-Verhältnis des Textilgewebes/- Bindemittels zwischen 1/10 und 1/15 liegt.

8. Verfahren nach Anspruch 6 oder 7, **dadurch gekennzeichnet, dass** die Temperatur zwischen 60 und 80°C, bevorzugter zwischen 65 und 75°C, liegt.

9. Verfahren nach irgendeinem der Ansprüche 6 bis 8, **dadurch gekennzeichnet, dass** mindestens ein Cyclodextrin und/oder ein Derivat davon in einer Konzentration zwischen 20 und 40 %, vorzugsweise zwischen 25 und 35 %, in einem geeigneten Lösungsmittel vorliegt, wobei das geeignete Lösungsmittel beispielsweise Dimethylsulfoxid ist.

10. Verfahren nach irgendeinem der Ansprüche 6 bis 9, **dadurch gekennzeichnet, dass** die Zeit in Schritt B zwischen 60 und 120 Minuten, vorzugsweise zwischen 80 und 100 Minuten, liegt.

11. Verfahren nach irgendeinem der Ansprüche 6 bis 10, **dadurch gekennzeichnet, dass** im Schritt B die Temperatur gleich oder größer als 50°C ist.

12. Textilgewebe, welches zur Absorption und Freisetzung eines Wirkstoffs geeignet ist, **dadurch gekennzeichnet, dass** es unter Verwendung des Bindemittels wie in irgendeinem der Ansprüche 1 bis 5 definiert und Durchführung der Verfahrensschritte wie in irgendeinem der Ansprüche 6 bis 11 definiert hergestellt wird.

13. Textilgewebe mit einem Wirkstoff, welcher bereit ist, in einer allmählichen und kontrollierten Weise freigesetzt zu werden, **dadurch gekennzeichnet, dass** es das Textilgewebe gemäß Anspruch 12 ist, welches mit irgendeinem der folgenden Verfahren behandelt wurde:
- Platzieren des Wirkstoffs in ein Bad bei Umgebungstemperatur und Halten in dem Bad unter leichter mechanischer Bewegung während einer Zeitspanne, die nicht kleiner als 30 Minuten ist, gefolgt von Trocknen;
oder
- Verstäuben der Lösung mit dem Wirkstoff auf das Textilgewebe und Abwarten für einige Minuten zu dessen Absorption.

14. Verwendung des Textilgewebes nach Anspruch 12 oder 13 für die kontrollierte Freisetzung von Wirkstoffen.

15. Verwendung des Textilgewebes nach Anspruch 14 für die intradermale kontrollierte Freisetzung von Wirkstoffen.

## Revendications

1. Composition de liant pour lier des cyclodextrines à des tissus, **caractérisée en ce qu'**il s'agit d'un mélange liquide comprenant de l'acide sulfamique selon un pourcentage en poids compris entre 0,8 et 1,2 par rapport audit mélange liquide, du sulfamate d'ammonium selon un pourcentage en poids compris entre 1,2 et 1,6 par rapport audit mélange liquide, et du sulfate d'ammonium selon un pourcentage en poids compris entre 2,8 et 3,2 par rapport audit mélange liquide.

2. Composition selon la revendication 1, **caractérisée en ce que** l'acide sulfamique est présent selon un pourcentage en poids compris entre 0,9 et 1,1 par rapport au mélange complet, le sulfate d'ammonium est présent selon un pourcentage en poids compris entre 1,3 et 1,5, et le sulfate d'ammonium est présent selon un pourcentage en poids compris entre 2,9 et 3,1 par rapport au mélange complet.

3. Composition selon la revendication 2, **caractérisée en ce que** l'acide sulfamique est présent selon un pourcentage en poids sensiblement égal à 1,0 par rapport au mélange complet, le sulfamate d'ammonium est présent selon un pourcentage en poids sensiblement égal à 1,5, et le sulfate d'ammonium est présent selon un pourcentage en poids sensiblement égal à 3,0 par rapport au mélange complet.

4. Composition selon l'une quelconque des revendications 1 à 3, **caractérisée en ce que** le mélange comprend de l'eau en tant que solvant.

5. Composition selon l'une quelconque des revendications 1 à 4, **caractérisée en ce que** ledit mélange liquide comprend des acides et des sels constitués par de l'acide sulfamique, du sulfamate d'ammonium et du sulfate d'ammonium.

6. Procédé de production d'un tissu apte à absorber et libérer un principe actif, **caractérisé en ce qu'**il utilise un liant et au moins une cyclodextrine et/ou un de ses dérivés, le liant étant le liant tel que défini dans l'une quelconque des revendications 1 à 5, le procédé étant en outre **caractérisé en ce qu'**il comprend les étapes suivantes :
A. mise en place du tissu dans un bain présentant un rapport tissu/liant non supérieur à 1/10 poids/volume, et à une température comprise dans la plage de 30-80°C ;
B. mise en place dans le bain d'au moins une cyclodextrine et/ou d'un de ses dérivés, et agitation constante de ce mélange pendant un laps de temps supérieur à 60 minutes à la même température que l'étape A ;
C. évacuation du bain et rinçage du tissu avec de l'eau froide ;
ledit laps de temps et ladite température dépendant de la composition des fibres constituant le tissu et du type de la cyclodextrine.

7. Procédé selon la revendication 6, **caractérisé en ce que** ledit rapport tissu/liant est compris entre 1/10 et 1/15 poids/volume.

8. Procédé selon la revendication 6 ou 7, **caractérisé en ce que** ladite température est comprise entre 60 et 80°C, plus particulièrement entre 65 et 75°C.

9. Procédé selon l'une quelconque des revendications 6 à 8, **caractérisé en ce qu'**au moins une cyclodextrine et/ou un de ses dérivés est présent selon une concentration comprise entre 20 et 40 % dans un solvant approprié, de préférence entre 25 et 35 %, ledit solvant approprié étant par exemple le diméthylsulfoxyde.

10. Procédé selon l'une quelconque des revendications 6 à 9, **caractérisé en ce que** dans l'étape B ledit laps de temps est compris entre 60 et 120 minutes, de préférence entre 80 et 100 minutes.

11. Procédé selon l'une quelconque des revendications 6 à 10, **caractérisé en ce que** dans l'étape B ladite température est supérieure ou égale à 50°C.

12. Tissu apte à absorber et libérer un principe actif, **caractérisé en ce qu'**il est produit par utilisation du liant tel que défini dans l'une quelconque des revendications 1 à 5 et mise en oeuvre des étapes du procédé telles que définies dans l'une quelconque des revendications 6 à 11.

13. Tissu comportant un principe actif prêt à être libéré d'une manière progressive et contrôlée, **caractérisé en ce qu'**il s'agit du tissu selon la revendication 12, traité par l'un quelconque des procédés suivants :
- mise en place du principe actif dans un bain à la température ambiante, et son maintien dans le bain sous légère agitation pendant un intervalle de temps non inférieur à 30 minutes, suivie d'un séchage ; ou
- atomisation de la solution comprenant le principe actif sur le tissu, attente de quelques minutes pour son absorption.

14. Utilisation du tissu selon la revendication 12 ou 13 pour la libération contrôlée de principes actifs.

15. Utilisation du tissu selon la revendication 14 pour la libération contrôlée intradermique de principes actifs.
